# EUROPEAN PATENT APPLICATION

(11) **EP 4 420 642 A1**
(43) Date of publication of application: **28.08.2024**
(21) Application number: 22883662.3
(22) Date of filing: 21.10.2022
(51) Int. Cl.: A61F 9/007, F21S 2/00, F21V 8/00, G02B 21/06, F21Y 101/00, F21Y 115/10

(54) **INTRAOCULAR ILLUMINATION DEVICE AND ATTACHMENT FOR INTRAOCULAR ILLUMINATION**

(30) Priority: 22.10.2021 JP 2021173128
(71) Applicant: University of Tsukuba, Ibaraki 305-8577 (JP); Teikyo University, Tokyo 173-8605 (JP)
(72) Inventor: MORIKAWA Shohei, Tsukuba-shi, Ibaraki 305-8577 (JP); MIHASHI Toshifumi, Tokyo 173-8605 (JP)
(74) Representative: Schwarz & Partner Patentanwälte GmbH
(86) International application number: PCT/JP2022/039240
(87) International publication number: WO 2023/068359

(57) **Abstract**

An intraocular illumination device includes a fiber that guides light from a light source, and a holder that is disposed between an objective lens of a microscope and an eye during eye surgery or examination and supports a tip portion of the fiber. The tip portion of the fiber is provided with a reflecting portion that reflects the light guided through the fiber toward an inside of the eye.

## Description

### Technical Field

The present disclosure relates to an intraocular illumination device and an intraocular illumination attachment.

### Background Art

Conventional intraocular illumination devices in ophthalmic surgery are inserted into a patient's eye to perform direct illumination. Therefore, it is necessary to create a port from the sclera into the eye, and there is a drawback that becomes a risk of infection accordingly. In addition, since one hand of an operator is blocked to operate an illumination device, surgical instrument can be normally operated only with one hand.

JP 2005-230558 A proposes a technique in which a light source is disposed at a position opposite to the eye with respect to an objective lens inside a microscope, and the inside of the eye is illuminated from the outside the eye with light passing through the objective lens. However, in the technique of JP 2005-230558 A, since the light source is disposed at a position opposite to the eye with respect to the objective lens and light enters the eye through the objective lens, it is difficult to obtain intraocular illumination having a sufficient light amount and an irradiation range. In addition, since the light source is disposed inside the microscope, it is necessary to newly purchase a microscope having such a special structure, and there is a disadvantage that an existing microscope cannot be used.

### Summary of Invention

A technique capable of providing intraocular illumination from outside the eye with a sufficient light amount and range is desired. In addition, a technique capable of realizing intraocular illumination from the outside the eye while using an existing microscope is desired.

An intraocular illumination device according to one aspect of the present disclosure including:
a fiber that guides light from a light source; and
a holder that is disposed between an objective lens of a microscope and an eye during eye surgery or examination and supports a tip portion of the fiber, in which
the tip portion of the fiber is provided with a reflecting portion that reflects the light guided through the fiber toward an inside of the eye.

An intraocular illumination attachment according to one aspect of the present disclosure is an intraocular illumination attachment disposed between an objective lens of a microscope and an eye during eye surgery or examination, the intraocular illumination attachment including:
an illumination unit attached to a tip portion of a fiber that guides light from a light source and including a mirror that reflects the light guided through the fiber into the eye; and
a holder that supports the illumination unit.

### Brief Description of Drawings

Fig. 1 is a schematic view illustrating a positional relationship between an intraocular illumination device, an objective lens of a microscope, and an eye according to an embodiment.
Fig. 2 is an enlarged perspective view of the intraocular illumination device.
Fig. 3 is a plan view of the intraocular illumination device as viewed from an eye side.
Fig. 4 is a perspective view of a holder of an intraocular illumination attachment as viewed from an objective lens side of a microscope.
Fig. 5 is a perspective view of the holder of the intraocular illumination attachment as viewed from the eye side.
Fig. 6 is an enlarged perspective view illustrating an illumination unit attached to a tip portion of a fiber.
Fig. 7A is a longitudinal sectional view of the illumination unit.
Fig. 7B is a perspective view of the illumination unit.
Fig. 8 is an eye fundus photograph taken when actual eye fundus observation is performed using the intraocular illumination device.
Fig. 9 is an enlarged perspective view illustrating an intraocular illumination device according to a modification.
Fig. 10 is a plan view of the intraocular illumination device according to the modification as viewed from an eye side.
Fig. 11 is a perspective view of a holder of an intraocular illumination attachment according to the modification as viewed from an objective lens side of a microscope.
Fig. 12 is a perspective view of the holder of the intraocular illumination attachment according to the modification as viewed from the eye side.
Fig. 13 is a schematic view illustrating a positional relationship between an intraocular illumination device, an objective lens of a microscope, and an eye according to the modification.
Fig. 14 is a schematic view illustrating a positional relationship between the intraocular illumination device, the objective lens of the microscope, and the eye according to the modification.
Fig. 15 is a view illustrating specific dimensions of the holder of the intraocular illumination device actually used in the embodiment.
Fig. 16 is a view illustrating specific dimensions of a cylindrical portion of an illumination unit of the intraocular illumination device actually used in the embodiment.

### Description of Embodiments

An intraocular illumination device according to a first aspect of the embodiment including:
a fiber that guides light from a light source; and
a holder that is disposed between an objective lens of a microscope and an eye during eye surgery or examination and supports a tip portion of the fiber, in which
the tip portion of the fiber is provided with a reflecting portion that reflects the light guided through the fiber toward an inside of the eye.

According to such an aspect, the tip portion of the fiber that guides light from the light source is supported by the holder between the objective lens of the microscope and the eye, and the tip portion of the fiber is provided with the reflecting portion that reflects the light guided through the fiber toward the inside of the eye. Therefore, the light can be emitted toward the inside of the eye from a position sufficiently close to the eye without passing through the lens. Therefore, it is possible to obtain intraocular illumination from the outside the eye with a sufficient light amount and range. In addition, since the tip portion of the fiber is supported by the holder so as not to move, it is possible for an operator to operate surgical instrument with both hands, that is, it is possible to practice a dual technique.

An intraocular illumination device according to a second aspect of the embodiment is the intraocular illumination device according to the first aspect, in which
an illumination unit including a mirror that reflects the light guided through the fiber toward the inside of the eye is attached to the tip portion of the fiber, and
the reflecting portion is configured by the mirror.

An intraocular illumination device according to a third aspect of the embodiment is the intraocular illumination device according to the first aspect, in which
an end surface of the tip portion of the fiber is obliquely polished so as to reflect the light guided through the fiber toward the eye, and
the reflecting portion is configured by the end surface of the fiber.

An intraocular illumination device according to a fourth aspect of the embodiment is the intraocular illumination device according to any one of the first to third aspects, in which
a thickness of the holder is 10 mm or less.

According to such an aspect, the holder can be easily disposed in a narrow space between the objective lens of the microscope and the eye.

An intraocular illumination device according to a fifth aspect of the embodiment is the intraocular illumination device according to any one of the first to fourth aspects, in which
the holder supports a plurality of the tip portions of the fibers.

According to such an aspect, since the holder supports the plurality of tip portions of the fibers, it is possible to increase the light amount and range of intraocular illumination.

An intraocular illumination device according to a sixth aspect of the embodiment is the intraocular illumination device according to the fifth aspect, in which
a plurality of the reflecting portions provided at each of the plurality of tip portions are disposed in the holder so as to be arranged at equal intervals along a circumferential direction about an optical axis of the microscope.

According to such an aspect, a wide range in the eye can be uniformly illuminated.

An intraocular illumination device according to a seventh aspect of the embodiment is the intraocular illumination device according to any one of the first to sixth aspects, in which
the holder is provided with an attachment portion attachable to a lens unit of the microscope.

An intraocular illumination device according to an eighth aspect of the embodiment is the intraocular illumination device according to any one of the first to sixth aspects, in which
the holder is configured integrally with a lens unit of the microscope.

An intraocular illumination device according to a ninth aspect of the embodiment is the intraocular illumination device according to any one of the first to eighth aspects, in which
a through-hole is formed in the holder coaxially with the optical axis of the microscope, a groove or hole is formed to extend outward from a vicinity of the through-hole in plan view, and the tip portion of the fiber is inserted into the groove or hole formed in the holder and supported.

According to such an aspect, since the through-hole is formed coaxially with the optical axis of the microscope in the holder, a bright field of view can be obtained when observed with the microscope. In addition, since the tip portion of the fiber is inserted into the groove or hole formed in the holder and supported, the configuration for supporting the tip portion of the fiber can be realized with a very simple structure, and the manufacturing cost of the holder can be reduced.

An intraocular illumination device according to a tenth aspect of the embodiment is the intraocular illumination device according to the ninth aspect, in which
the tip portion of the fiber is positionally adjustable along the groove or hole in a state of being supported by the holder.

According to such an aspect, by adjusting a position of the tip portion of the fiber, brightness of an eye fundus and a condition of a spot (illuminated portion) can be easily adjusted.

An intraocular illumination device according to an eleventh aspect of the embodiment is the intraocular illumination device according to the tenth aspect, in which the tip portion of the fiber is positionally adjusted along the groove or hole, so that the reflecting portion can be brought close to a range within 5 mm from the optical axis of the microscope.

According to such an aspect, while a diameter of the iris (cornea) is usually 10 to 12 mm, a window portion can be brought close to a range within 5 mm from the optical axis of the microscope, so that light emitted from the window portion can be efficiently introduced into the eye from the portion of the iris.

An intraocular illumination device according to a twelfth aspect of the embodiment is the intraocular illumination device according to any one of the ninth to eleventh aspects, in which
a number of the tip portions of the fibers is four, the holder is formed with four of the grooves or holes, and the four grooves or holes are formed to extend in directions of 0°, 90°, 180°, and 270°, respectively, in polar coordinates about the optical axis of the microscope.

An intraocular illumination device according to a thirteenth aspect of the embodiment is the intraocular illumination device according to any one of the ninth to eleventh aspects, in which
a number of the tip portions of the fibers is four, the holder is formed with four of the grooves or holes, a first groove or hole is formed to extend in a direction of 0° from a position of 0°, a second groove or hole is formed to extend in a direction of 180° from a position of 180°, a third groove or hole is formed to extend in a direction of 0° or 180° from a position of 90°, and a fourth groove or hole is formed to extend in a direction of 0° or 180° from a position of 270° in polar coordinates about the optical axis of the microscope.

An intraocular illumination device according to a fourteenth aspect of the embodiment is the intraocular illumination device according to any one of the first to thirteenth aspects, in which
an NA of the fiber on an emitting side is 0.55 or less.

According to such an aspect, it is possible to emit light having sufficient directionality toward the inside of the eye, and it is possible to increase the light amount and range of intraocular illumination.

An intraocular illumination device according to a fifteenth aspect of the embodiment is the intraocular illumination device according to any one of the first to fourteenth aspects, in which
the fiber is branched between the light source and the illumination unit.

According to such an aspect, a number of illumination units can be increased without increasing a number of light sources.

An intraocular illumination device according to a sixteenth aspect of the embodiment is the intraocular illumination device according to any one of the first to fifteenth aspects, in which
the light source is at least one of a xenon lamp, a halogen lamp, and an LED.

According to such an aspect, it is possible to use a xenon lamp, a halogen lamp, or an LED existing in an operating room or an inspection room as the light source, and it is convenient since it is not necessary to newly prepare a light source.

An intraocular illumination attachment according to a seventeenth aspect of the embodiment is an intraocular illumination attachment disposed between an objective lens of a microscope and an eye during eye surgery or examination, the intraocular illumination attachment including:
an illumination unit attached to a tip portion of a fiber that guides light from a light source and including a mirror that reflects the light guided through the fiber into the eye; and
a holder that supports the illumination unit.

According to such an aspect, since the intraocular illumination attachment is disposed between the objective lens of the microscope and the eye during eye surgery or examination, that is, disposed outside the microscope, intraocular illumination from outside the eye can be realized while utilizing the existing microscope. In addition, since the illumination unit including the mirror that reflects the light guided through the fiber toward the inside of the eye is supported by the holder between the objective lens of the microscope and the eye, the light can be emitted toward the inside of the eye from a position sufficiently close to the eye without passing through the lens. Therefore, it is possible to obtain intraocular illumination from the outside the eye with a sufficient light amount and range. In addition, since the illumination unit is supported by the holder so as not to move, it is possible for an operator to operate the surgical instrument with both hands, that is, it is possible to practice the dual technique.

An intraocular illumination attachment according to an eighteenth aspect of the embodiment is the intraocular illumination attachment according to the seventeenth aspect, in which
a thickness of the holder is 10 mm or less.

According to such an aspect, the holder can be easily disposed in a narrow space between the objective lens of the microscope and the eye.

An intraocular illumination attachment according to a nineteenth aspect of the embodiment is the intraocular illumination attachment according to the seventeenth or eighteenth aspect, in which
the holder supports a plurality of the illumination units.

According to such an aspect, since the holder supports the plurality of illumination units, the light amount and range of intraocular illumination can be increased.

An intraocular illumination attachment according to a twentieth aspect of the embodiment is the intraocular illumination attachment according to the nineteenth aspect, in which
the plurality of illumination units are disposed such that window portions through which light reflected by the mirror is emitted are arranged at equal intervals along a circumferential direction about the optical axis of the microscope.

According to such an aspect, a wide range in the eye can be uniformly illuminated.

An intraocular illumination attachment according to a 21th aspect of the embodiment is the intraocular illumination attachment according to any one of the seventeenth to twentieth aspects, in which
the holder is provided with an attachment portion attachable to a lens unit of the microscope.

According to such an aspect, the holder can be easily fixed so as not to move with respect to the lens unit.

An intraocular illumination attachment according to a 22nd aspect of the embodiment is the intraocular illumination attachment according to any one of the seventeenth to 21th aspects, in which
a through-hole is formed in the holder coaxially with an optical axis of the microscope, a groove or hole is formed to extend outward from a vicinity of the through-hole in plan view, and the illumination unit attached to the tip portion of the fiber is inserted into the groove or hole formed in the holder and supported.

According to such an aspect, since the through-hole is formed coaxially with the optical axis of the microscope in the holder, a bright field of view can be obtained when observed with the microscope. In addition, since the illumination unit is inserted into the groove or hole formed in the holder and supported, the configuration for supporting the illumination unit can be realized with a very simple structure, and the manufacturing cost of the holder can be reduced.

An intraocular illumination attachment according to a 23rd aspect of the embodiment is the intraocular illumination attachment according to the 22nd aspect, in which it is the intraocular illumination attachment according to claim 6, in which
the illumination unit attached to the tip portion of the fiber is positionally adjustable along the groove or hole in a state of being supported by the holder.

According to such an aspect, by adjusting the position of the illumination unit, brightness of an eye fundus and a condition of a spot (illuminated portion) can be easily adjusted.

An intraocular illumination attachment according to a 24th aspect of the embodiment is the intraocular illumination attachment according to the 23th aspect, in which
the illumination unit attached to the tip portion of the fiber is positionally adjusted along the groove or hole, so that the window portion that emits the light reflected by the mirror can be brought close to a range within 5 mm from the optical axis of the microscope.

According to such an aspect, while a diameter of the iris is usually 10 to 12 mm, a window portion can be brought close to the range within 5 mm from the optical axis of the microscope, so that light emitted from the window portion can be efficiently introduced into the eye from the portion of the iris.

An intraocular illumination attachment according to a 25th aspect of the embodiment is the intraocular illumination attachment according to any one of the 22nd to 24th aspects, in which
a number of the illumination units is four, the holder is formed with four of the grooves or holes, and the four grooves or holes are formed to extend in directions of 0°, 90°, 180°, and 270°, respectively, in polar coordinates about the optical axis of the microscope.

An intraocular illumination attachment according to a 26th aspect of the embodiment is the intraocular illumination attachment according to any one of the 22nd to 24th aspects, in which
a number of the illumination units is four, the holder is formed with four of the grooves or holes, a first groove or hole is formed to extend in a direction of 0° from a position of 0°, a second groove or hole is formed to extend in a direction of 180° from a position of 180°, a third groove or hole is formed to extend in a direction of 0° or 180° from a position of 90°, and a fourth groove or hole is formed to extend in a direction of 0° or 180° from a position of 270° in polar coordinates about the optical axis of the microscope.

An intraocular illumination attachment according to a 27th aspect of the embodiment is the intraocular illumination attachment according to any one of the seventeenth to 26th aspects, in which
the intraocular illumination attachment is disposable.

According to such an aspect, the intraocular illumination attachment is disposed between the objective lens of the microscope and the eye during eye surgery or examination, so that the intraocular illumination attachment is easily contaminated. However, since the intraocular illumination attachment is disposable, the intraocular illumination attachment is extremely hygienic and convenient.

An intraocular illumination attachment according to a 28th aspect of the embodiment is the intraocular illumination attachment according to any one of the seventeenth to 27th aspects, in which
an NA of the fiber on an emitting side is 0.55 or less.

According to such an aspect, it is possible to emit light having sufficient directionality toward the inside of the eye, and it is possible to increase the light amount and range of intraocular illumination.

An intraocular illumination attachment according to a 29th aspect of the embodiment is the intraocular illumination attachment according to any one of the seventeenth to 28th aspects, in which
the fiber is branched between the light source 15 and the illumination unit.

According to such an aspect, a number of illumination units can be increased without increasing a number of light sources.

An intraocular illumination attachment according to a 30th aspect of the embodiment is the intraocular illumination attachment according to any one of the seventeenth to 29th aspects, in which
the light source is at least one of a xenon lamp, a halogen lamp, and an LED.

According to such an aspect, it is possible to use a xenon lamp, a halogen lamp, or an LED existing in an operating room or an inspection room as the light source, and it is convenient since it is not necessary to newly prepare a light source.

An intraocular illumination device according to a 31st aspect of the embodiment including:
the intraocular illumination attachment according to any one of the seventeenth to 30th aspects; and the fiber.

A microscope for eye surgery or examination according to a 32nd aspect of the embodiment including:
the intraocular illumination device according to any one of the first to sixteenth and 31th aspects.

Hereinafter, specific examples of the embodiment will be described in detail with reference to the accompanying drawings. Note that, in the respective drawings, components having equivalent functions are denoted by the same reference numerals, and detailed description of the components having the same reference numerals will not be repeated.

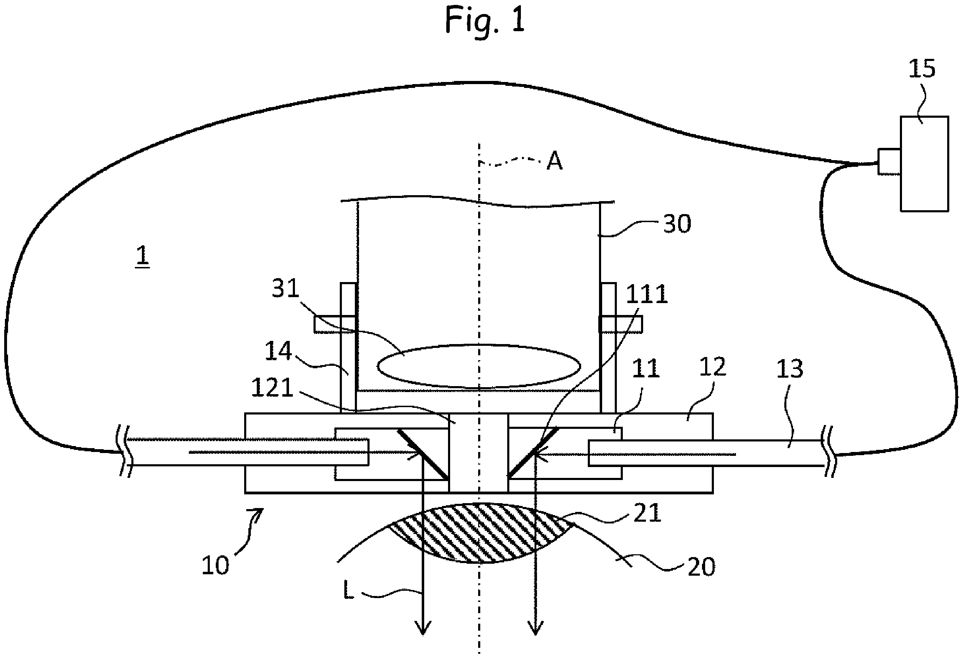

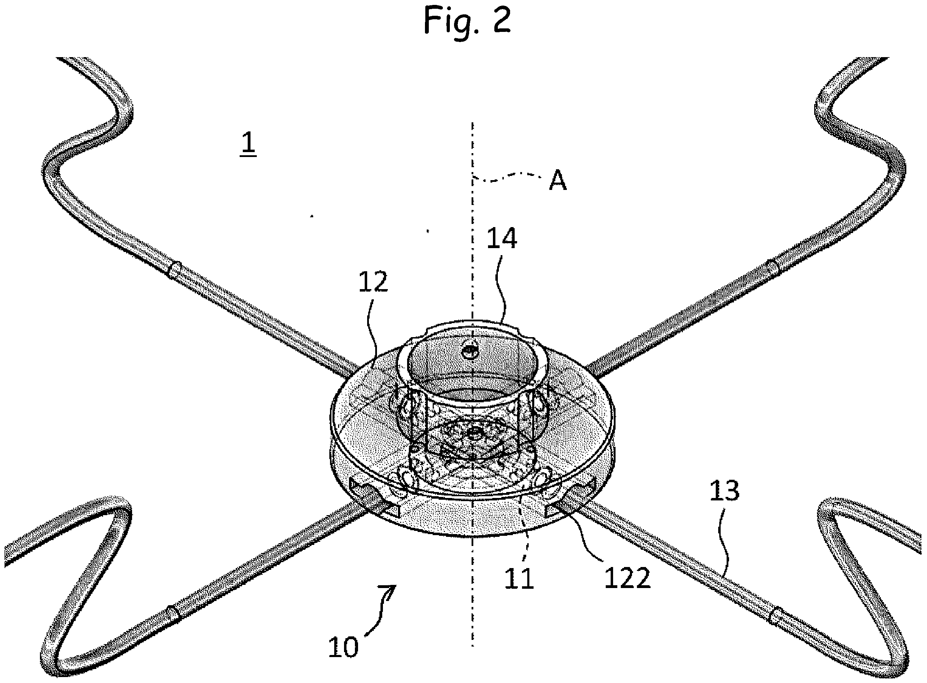

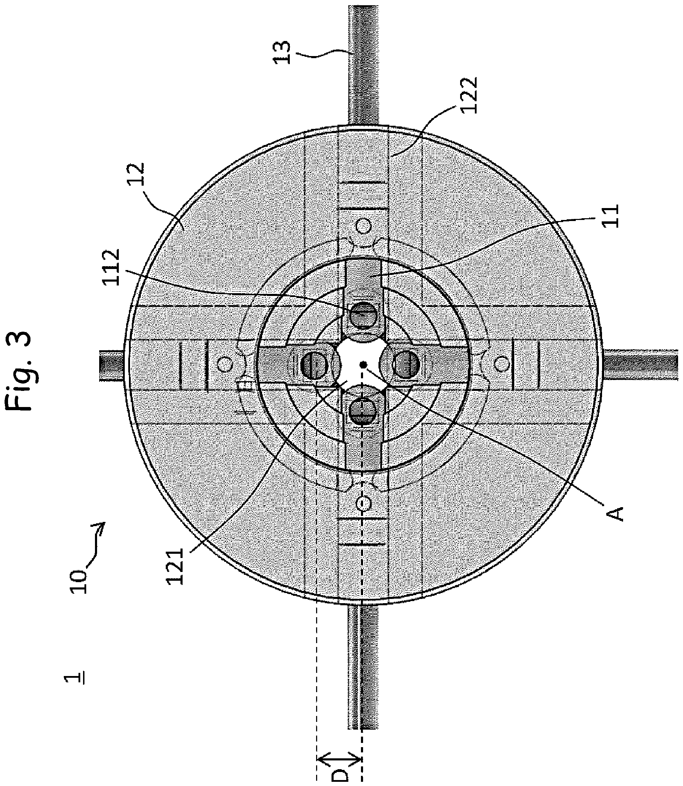

Fig. 1 is a schematic view illustrating a positional relationship between an intraocular illumination device 1 according to an embodiment, an objective lens 31 of a microscope, and an eye 20. Fig. 2 is an enlarged perspective view of the intraocular illumination device 1. Fig. 3 is a plan view of the intraocular illumination device 1 as viewed from the eye 20 side.

As illustrated in Figs. 1 to 3, the intraocular illumination device 1 has fibers (optical fibers) 13 that guide light from a light source 15, and an intraocular illumination attachment 10 attached to tip portions of the fibers 13.

Among them, as illustrated in Fig. 1, the intraocular illumination attachment 10 is disposed between the objective lens 31 of the microscope and the eye 20 during surgery or examination of the eye 20. Since the intraocular illumination attachment 10 is disposed between the objective lens 31 and the eye 20 and is easily contaminated, it may be disposable in consideration of hygiene. Note that the objective lens 31 of the microscope is accommodated in the lens unit 30.

As illustrated in Figs. 1 to 3, the intraocular illumination attachment 10 has illumination units 11 attached to the tip portions of the fibers 13 and a holder 12 that supports the illumination units 11.

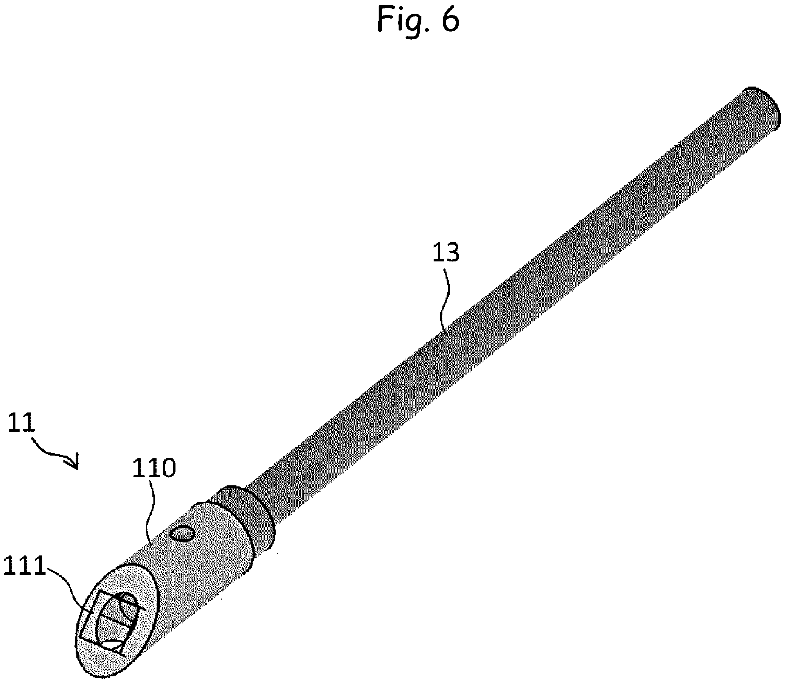

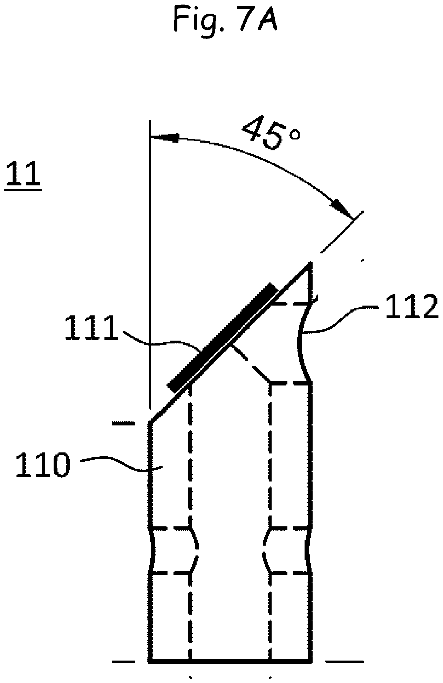

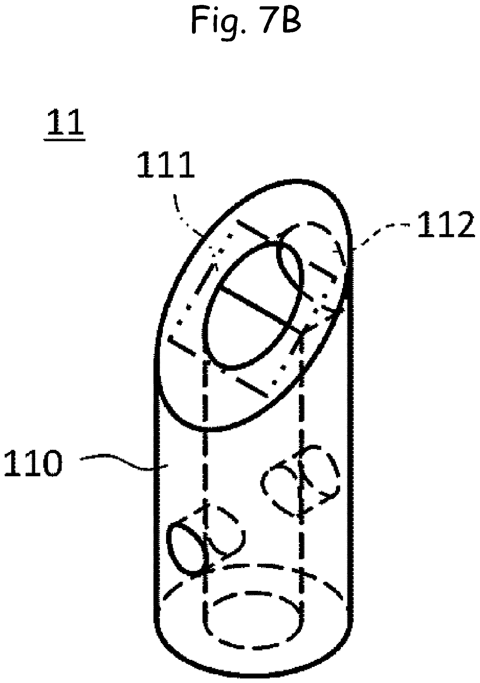

First, the structure of the illumination unit 11 will be described. Fig. 6 is an enlarged perspective view illustrating the illumination unit 11 attached to the tip portion of the fiber 13. Fig. 7A is a longitudinal sectional view of the illumination unit 11, and Fig. 7B is a perspective view of the illumination unit 11.

As illustrated in Figs. 6, 7A, and 7B, the illumination unit 11 has a cylindrical portion 110 having a cylindrical shape in which a tip portion is obliquely cut (for example, at an angle of 45°), and a mirror 111 attached to cover an opening exposed on a cut surface of the cylindrical portion 110. A material of the cylindrical portion 110 is, for example, brass. A window portion 112 is formed on an outer peripheral surface of the cylindrical portion 110 so as to correspond to a reflecting surface of the mirror 111.

As illustrated in Fig. 6, the tip portion of the fiber 13 is fixed to the cylindrical portion 110 in a state of being inserted from a proximal end portion of the cylindrical portion 110. The light emitted from the tip portion of the fiber 13 is reflected by the reflecting surface of the mirror 111 in a direction of 90° and then emitted to the outside through the window portion 112. That is, in the present embodiment, the reflecting portion that is provided at the tip portion of the fiber 13 and reflects the light guided through the fiber 13 toward the inside of the eye 20 is configured by the mirror 111 of the illumination unit 11.

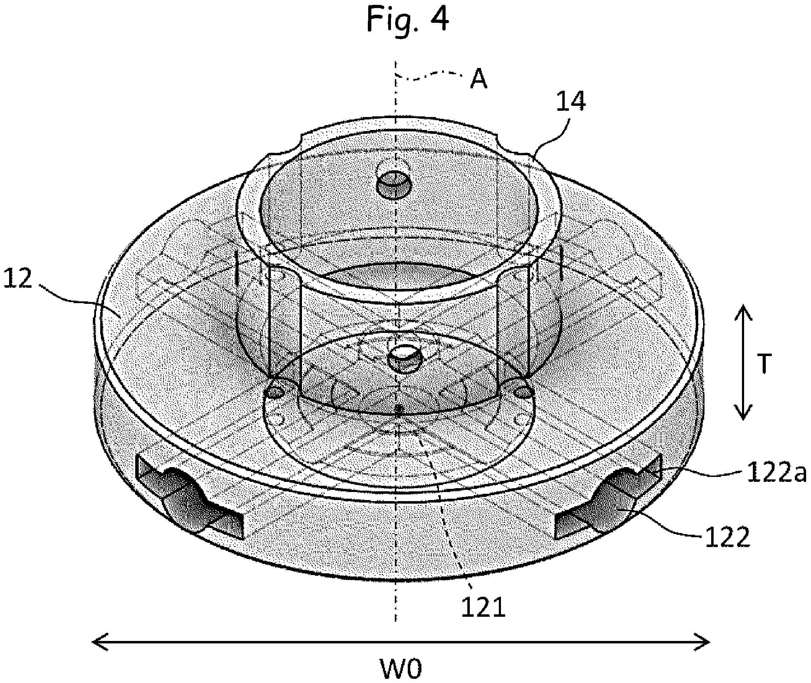

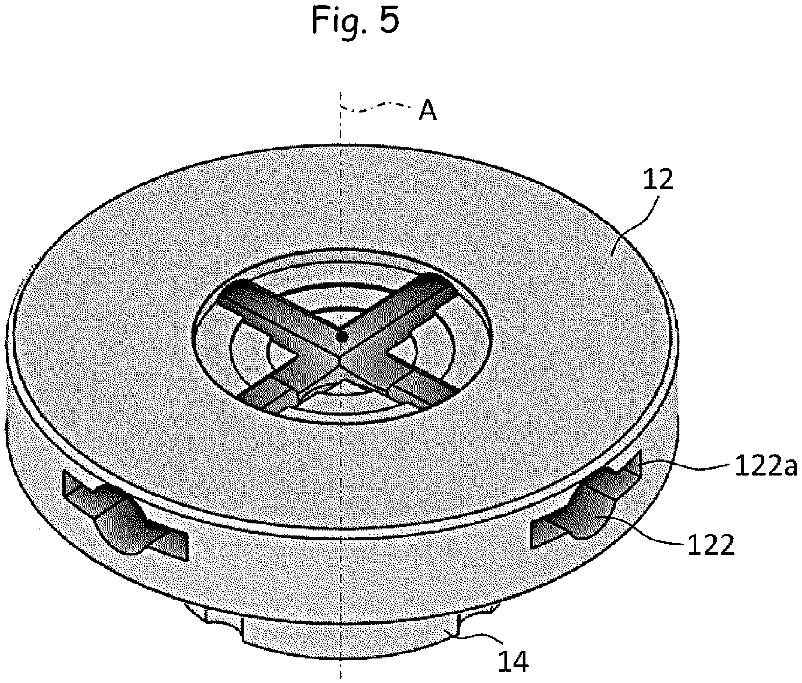

Next, the structure of the holder 12 will be described. Fig. 4 is a perspective view of the holder 12 as viewed from the objective lens 31 side of the microscope. Fig. 5 is a perspective view of the holder 12 as viewed from the eye 20 side.

As illustrated in Figs. 4 and 5, the holder 12 has a substantially disk shape. A thickness T (length of the microscope in a direction of an optical axis A) of the holder 12 may be, for example, 10 mm or less, 9 mm or less, or 8 mm or less. Since the thickness of the holder 12 is sufficiently thin, the holder 12 can be easily disposed in a narrow space between an objective lens 23 of the microscope and the eye 20 during surgery or examination of the eye 20. A diameter WO of the holder 12 may be, for example, 40 to 50 mm. A material of the holder 12 is, for example, a resin.

As illustrated in Figs. 4 and 5, the holder 12 is provided with an attachment portion 14 attachable to the lens unit 30 of the microscope. In the illustrated example, the attachment portion 14 has a substantially cylindrical shape, and is provided on the surface of the holder 12 coaxially with the optical axis A of the microscope. A diameter of the attachment portion 14 is, for example, 20 to 25 mm, and a height of the attachment portion 14 is, for example, 10 to 15 mm.

A screw hole is formed in an outer peripheral surface of the attachment portion 14. As illustrated in Fig. 1, the holder 12 is fixed so as not to move with respect to the lens unit 30 by tightening a screw inserted into the screw hole in a state where the lens unit 30 of the microscope is inserted into the attachment portion 14.

As illustrated in Figs. 3 and 4, a through-hole 121 is formed in the holder 12 coaxially with the optical axis A of the microscope. As a result, since the periphery of the optical axis A of the microscope is not blocked by the holder 12, a bright field of view can be obtained when observed with the microscope.

As illustrated in Figs. 3 to 5, grooves or holes 122 are formed in the holder 12 to extend outward from the vicinity of the through-hole 121 in plan view. Here, the "groove or hole" refers to a structure in which a portion near the through-hole 121 has a groove shape (a contour line of a cross section is open), but a portion away from the through-hole 121 has a hole shape (a contour line of a cross section is closed). A diameter of the groove or hole 122 is substantially the same as a diameter of the cylindrical portion 110 of the illumination unit 11, and the illumination unit 11 attached to the tip portion of the fiber 13 is configured to be supported by being inserted into the groove or hole 122 formed in the holder 12. As a result, the configuration for supporting the illumination unit 11 can be realized with a very simple structure, and the manufacturing cost of the holder 12 can be reduced.

By moving the fiber 13 in its axial direction, the illumination unit 11 attached to the tip portion of the fiber 13 can be positionally adjusted along the groove or hole 122 while being supported by the holder 12. By adjusting the position of the illumination unit 11, brightness of an eye fundus and a condition of a spot (illuminated portion) can be easily adjusted.

The illumination unit 11 attached to the tip portion of the fiber 13 is positionally adjusted along the groove or hole 122, so that the window portion 112 that emits the light reflected by the mirror 111 can be brought close to the optical axis A of the microscope. With reference to Fig. 3, an interval D between the optical axis A of the microscope and the window portion 112 may be brought close to a range within, for example, 5 mm or less. While the diameter of the iris 21 of the eye 20 is usually 10 to 12 mm, by bringing the window portion 112 close to the range within 5 mm from the optical axis A of the microscope, light emitted from the window portion 112 can be efficiently introduced into the eye 20 from the portion of the iris 21.

As illustrated in Figs. 4 and 5, a small groove 122a for rotation restriction may be formed adjacent to the groove or hole 122 for inserting the illumination unit 11. A projection (not illustrated) corresponding to the small groove 122a is provided on the outer peripheral surface of the cylindrical portion 110 of the illumination unit 11, and when the cylindrical portion 110 of the illumination unit 11 is inserted into the groove or hole 122 of the holder 12, the projection of the cylindrical portion 110 is inserted along the small groove 122a, so that the illumination unit 11 can be prevented from rotating about the axis of the fiber 13, and the direction of light emitted from the window portion 112 can be fixed so as not to move.

Only one groove or hole 122 may be formed in the holder 12 to support only one illumination unit 11, or a plurality of grooves or holes 122 may be formed to support a plurality of illumination units 11.

In the example illustrated in Figs. 2 and 3, four grooves or holes 122 are formed in the holder 12 so as to support the four illumination units 11, but the present disclosure is not limited thereto. For example, six or eight grooves or holes may be formed in the holder 12 so as to support six or eight illumination units 11. Since the holder 12 supports the plurality of illumination units 11, it is possible to increase the light amount and range of the intraocular illumination.

As illustrated in Figs. 2 and 3, the plurality of illumination units 11 may be disposed such that the window portions 112 that emit the light reflected by the mirror 111 are arranged at equal intervals along the circumferential direction about the optical axis A of the microscope. In the illustrated example, the four illumination units 11 are arranged at positions of 0°, 90°, 180°, and 270° in polar coordinates about the optical axis A of the microscope. By disposing the window portions 112 through which light is emitted at equal intervals in the circumferential direction, it is possible to uniformly illuminate a wide range in the eye.

In the present exemplary embodiment, as illustrated in Figs. 2 to 5, four grooves or holes 122 are formed in the holder 12 to extend in the directions of 0°, 90°, 180°, and 270°, respectively, in the polar coordinates about the optical axis A of the microscope. The four fibers 13 each having the illumination unit 11 attached to the tip portion are inserted into the grooves or holes 122 from different directions of 0°, 90°, 180°, and 270° and supported.

As illustrated in Fig. 1, in a state in which the holder 12 is disposed between the objective lens 31 of the microscope and the eye 20 and the illumination unit 11 attached to the tip portion of the fiber 13 is inserted into the groove or hole 122 of the holder 12 and supported, the window portion 112 of the illumination unit 11 is oriented to face the inside of the eye 20 (see Fig. 3), and light L emitted from the tip portion of the fiber 13 is reflected toward the inside of the eye 20 by the mirror 111 of the illumination unit 11. The illuminance of the light L emitted from the illumination unit 11 toward the inside of the eye 20 can be appropriately set according to the microscope to be used, but for example, the central illuminance at the eye fundus may be 10000 to 100000 lx. The illuminance is defined according to JIS Z 9110:2010.

A numerical aperture (NA) of the fiber 13 on the emitting side may be 0.55 or less, 0.50 or less, 0.45 or less, or 0.40 or less. Here, the numerical aperture (NA) is a value represented by nsinθ when an angle of a vertex of a maximum conical light beam emitted from a core of the fiber 13 is 2θ and a refractive index of a medium in which the fiber 13 is present is n, and is according to the definition of JIS C 6820:2018. Since the numerical aperture (NA) of the fiber 13 is small, it is possible to emit light having sufficient directionality toward the inside of the eye, and thus, it is possible to increase the light amount and range of the intraocular illumination.

As illustrated in Fig. 1, when the holder 12 supports the plurality of illumination units 11, the fiber 13 may be branched into a plurality of fibers between the light source 15 and each illumination unit 11. For example, in a case where the holder 12 supports four illumination units 11, one fiber 13 extending from one light source 15 may be branched into four and then coupled to four illumination units 11, or one fiber 13 extending from one light source 15 may be branched into two and then coupled to two illumination units 11, and another fiber 13 extending from another light source 15 may be branched into two and then coupled to remaining two illumination units 11. By branching the fiber 13 between the light source 15 and the illumination unit 11, the number of illumination units 11 can be increased without increasing the number of the light sources 15.

The light source 15 may be at least one of a xenon lamp, a halogen lamp, and an LED (light emitting diode). In this case, a xenon lamp, a halogen lamp, or an LED existing in an operating room or an inspection room can be used as the light source 15, and it is convenient since it is not necessary to newly prepare a light source.

According to the present embodiment as described above, since the intraocular illumination attachment 10 is disposed between the objective lens 31 of the microscope and the eye 20 during surgery or examination of the eye 20, that is, disposed outside the microscope, intraocular illumination from the outside of the eye can be realized using the existing microscope.

In addition, according to the present embodiment, since the illumination unit 11 including the mirror 111 that reflects the light guided through the fiber 13 toward the inside of the eye is supported by the holder 12 between the objective lens 31 of the microscope and the eye 20, the light can be emitted toward the inside of the eye from a position sufficiently close to the eye 20 without passing through the lens. Therefore, it is possible to obtain intraocular illumination from the outside the eye with a sufficient light amount and range.

In addition, according to the present embodiment, since the illumination unit 11 is supported by the holder 12 so as not to move, it is possible for an operator to operate the surgical instrument with both hands, that is, it is possible to practice the dual technique.

Note that, in the above-described embodiment, the holder 12 is provided with the attachment portion 14, and the holder 12 is fixed so as not to move with respect to the lens unit 30 of the microscope via the attachment portion 14. However, the present disclosure is not limited to such an aspect. For example, the holder 12 may not be provided with the attachment portion 14, and the holder 12 may be supported so as not to move with respect to the lens unit 30 using a support jig (for example, a stand with a clamp) (not illustrated).

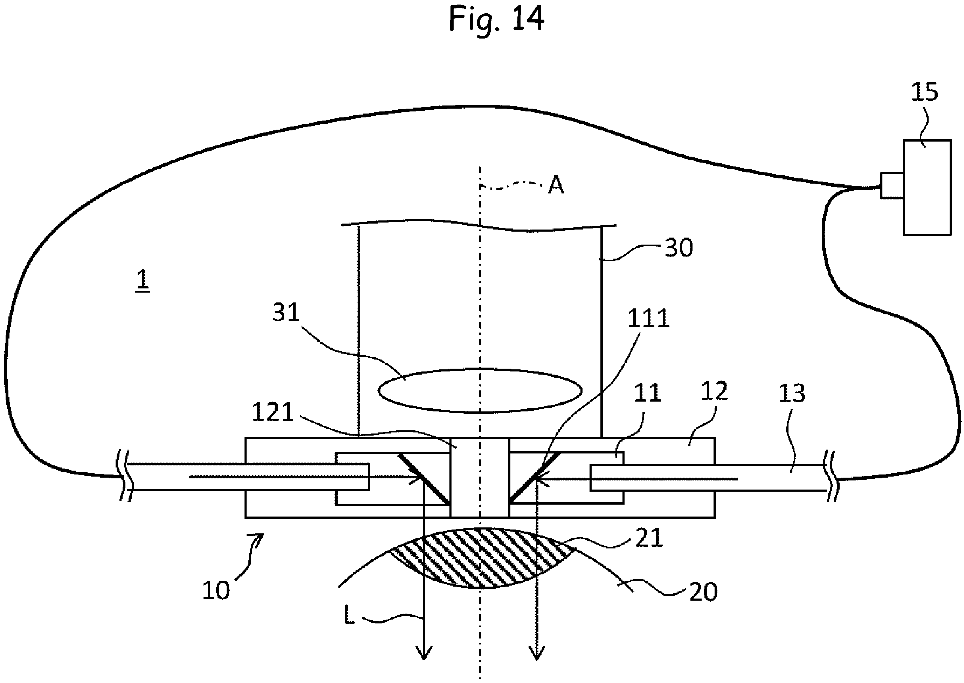

As a modification, as illustrated in Fig. 14, the holder 12 may be configured integrally with the lens unit 30 of the microscope (non-detachable with respect to the lens unit 30). In the example illustrated in Fig. 14, the holder 12 is disposed adjacent to the outside the lens unit 30, but the present disclosure is not limited thereto, and the holder 12 may be disposed inside the lens unit 30 as long as it is located between the objective lens 31 and the eye 20.

In addition, in the above-described embodiment, as illustrated in Figs. 2 to 5, in the holder 12, the four grooves or holes 122 are formed to extend in the directions of 0°, 90°, 180°, and 270°, respectively, in the polar coordinates about the optical axis A of the microscope, and the four fibers 13 to each of which the illumination unit 11 is attached at the tip portion are inserted into the grooves or holes 122 from the directions different from each other of 0°, 90°, 180°, and 270° and supported. However, the present disclosure is not limited to such an aspect.

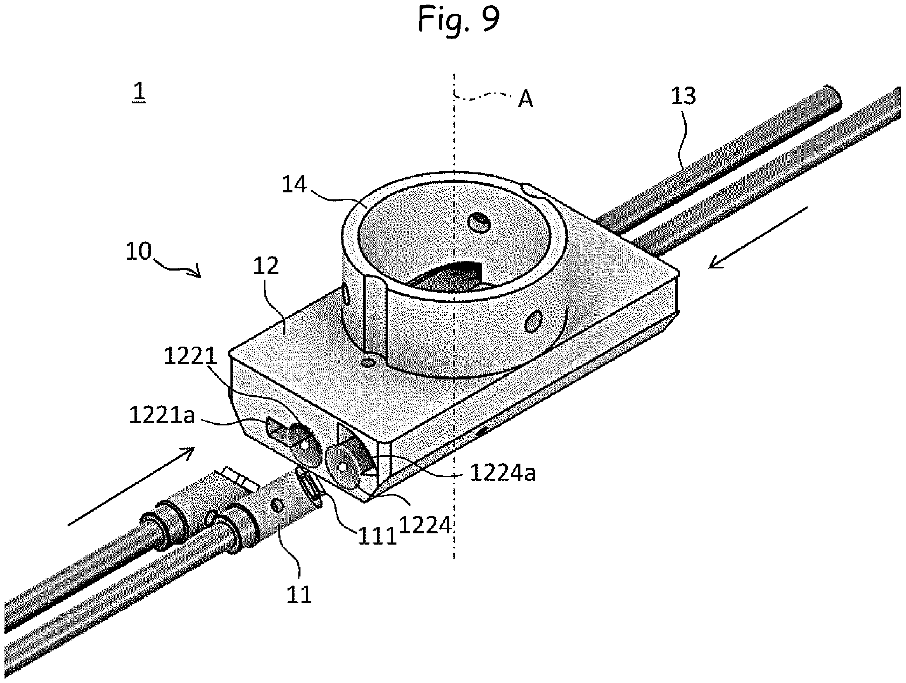

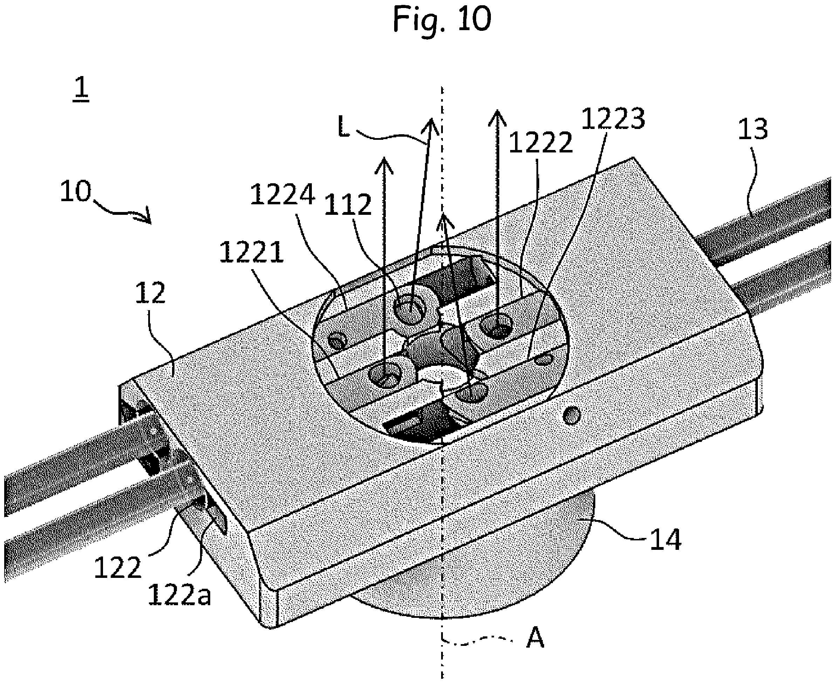

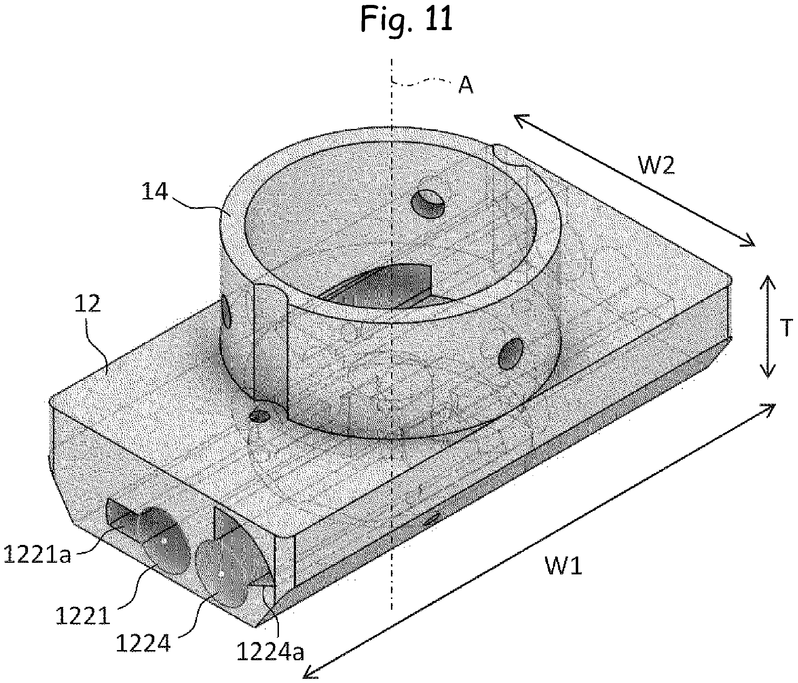

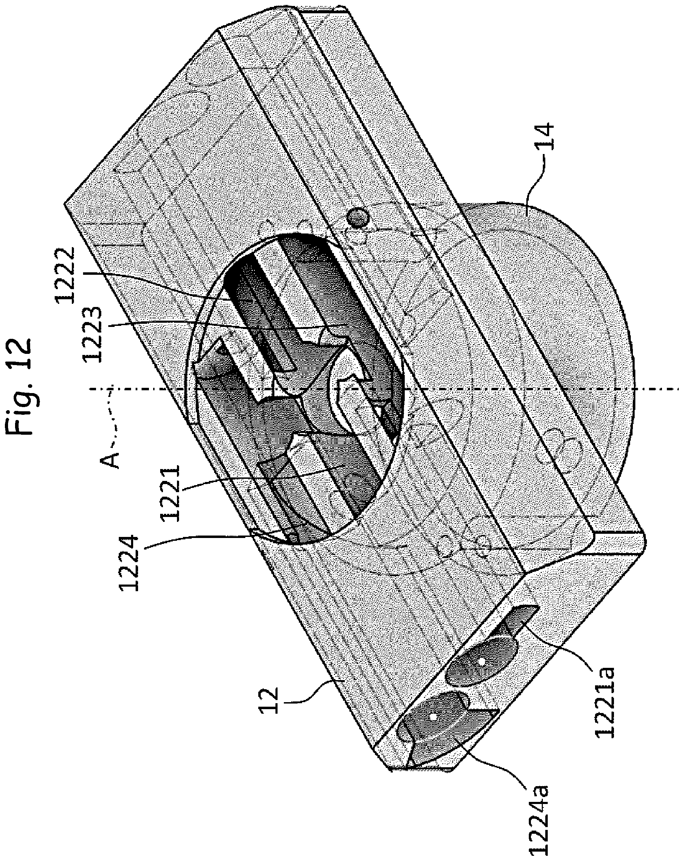

Fig. 9 is an enlarged perspective view illustrating the intraocular illumination device 1 according to the modification, and Fig. 10 is a plan view of the intraocular illumination device 1 as viewed from the eye 20 side. Fig. 11 is a perspective view of the holder 12 of the intraocular illumination attachment 10 according to the modification as viewed from the objective lens 31 side of the microscope. Fig. 12 is a perspective view of the holder 12 of the intraocular illumination attachment 10 according to the modification as viewed from the eye 20 side.

In the aspects illustrated in Figs. 9 to 12, the holder 12 has a substantially rectangular parallelepiped shape. The thickness T (length of the microscope in the direction of the optical axis A) of the holder 12 may be, for example, 10 mm or less, 9 mm or less, or 8 mm or less. Since the thickness of the holder 12 is sufficiently thin, the holder 12 can be easily disposed in a narrow space between an objective lens 23 of the microscope and the eye 20 during surgery or examination of the eye 20. A length W1 of the holder 12 in a long side direction may be, for example, 40 to 50 mm, and a length W2 in a short side direction may be, for example, 20 to 30 mm. The material of the holder 12 is, for example, a resin.

As illustrated in Figs. 10 and 12, four grooves or holes 1221 to 1224 for supporting the four illumination units 11 are formed in the holder 12.

In the polar coordinates about the optical axis A of the microscope, when the long side direction of the holder 12 is a direction of 0° and 180°, and the short side direction of the holder 12 is a direction of 90° and 270°, the first groove or hole 1221 is formed to extend in a direction of 0° from a position of 0°, and the second groove or hole 1222 is formed to extend in a direction of 180° from a position of 180°. Here,

On the other hand, the third groove or hole 1223 is formed to extend in a direction of 180° from a position of 90° (that is, parallel to the first groove or hole 1221) and the fourth groove or hole 1224 is formed to extend in a direction of 0° from a position of 270° (that is, parallel to the second groove or hole 1222).

Therefore, as illustrated in Fig. 9, among the four fibers 13 having the illumination units 11 attached to the tip portion, two fibers 13 are inserted into and supported by the first and fourth grooves or holes 1221 and 1224 from the same direction of 0°, and the remaining two fibers 13 are inserted into and supported by the second and third grooves or holes 1222 and 1223 from the same direction of 180°.

According to an aspect according to such a modification, the fiber 13 only needs to be routed from two directions of 0° and 180°, and does not need to be routed from the four directions. Therefore, the wiring of the fiber 13 can be made compact, and it is possible to suppress the presence of the fiber 13 from being an obstacle at the time of eye surgery or examination.

In addition, according to the aspect according to such a modification, the length W2 of the holder 12 in the short side direction can be reduced. As a result, the length W2 of the holder 12 in the short side direction can be set to a size that falls within a recess between the eyebrow and the cheek of the patient, and it becomes easier to dispose the holder 12 in a narrow space between the objective lens 31 of the microscope and the eye 20 during surgery or examination of the eye 20.

As illustrated in Figs. 9 to 12, a small groove 1224a for rotation restriction formed adjacent to the fourth groove or hole 1224 may have a cross-sectional fan shape. In this case, in a state where the illumination unit 11 attached to the tip portion of the fiber 13 is inserted into and supported by the fourth groove or hole 1224, the illumination unit 11 can be rotated by a minute angle (for example, 5° to 10°) about the axis of the fiber 13, and as illustrated in Fig. 10, the direction of the light L emitted from the window portion 112 of the illumination unit 11 can be adjusted to adjust the brightness of the eye fundus and the condition of the spot (illuminated portion).

Similarly, a small groove (not illustrated) for rotation restriction formed adjacent to the third groove or hole 1223 may also have a cross-sectional fan shape. In this case, in a state where the illumination unit 11 attached to the tip portion of the fiber 13 is inserted into and supported by the third groove or hole 1223, the illumination unit 11 can be rotated by a minute angle (for example, 5° to 10°) about the axis of the fiber 13, and as illustrated in Fig. 10, the direction of the light L emitted from the window portion 112 of the illumination unit 11 can be adjusted to adjust the brightness of the eye fundus and the condition of the spot (illuminated portion).

In the aspects illustrated in Figs. 9 to 12, the third groove or hole 1223 may be formed to extend in the direction of 0° (that is, parallel to the second groove or hole 1222) from the position of 90°, and the fourth groove or hole 1224 may be formed to extend in the direction of 180° (that is, parallel to the first groove or hole 1221) from the position of 270°.

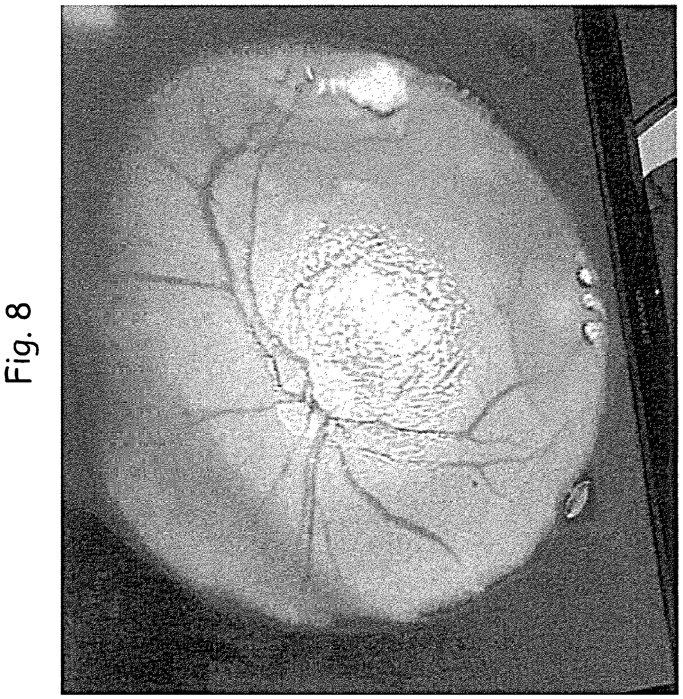

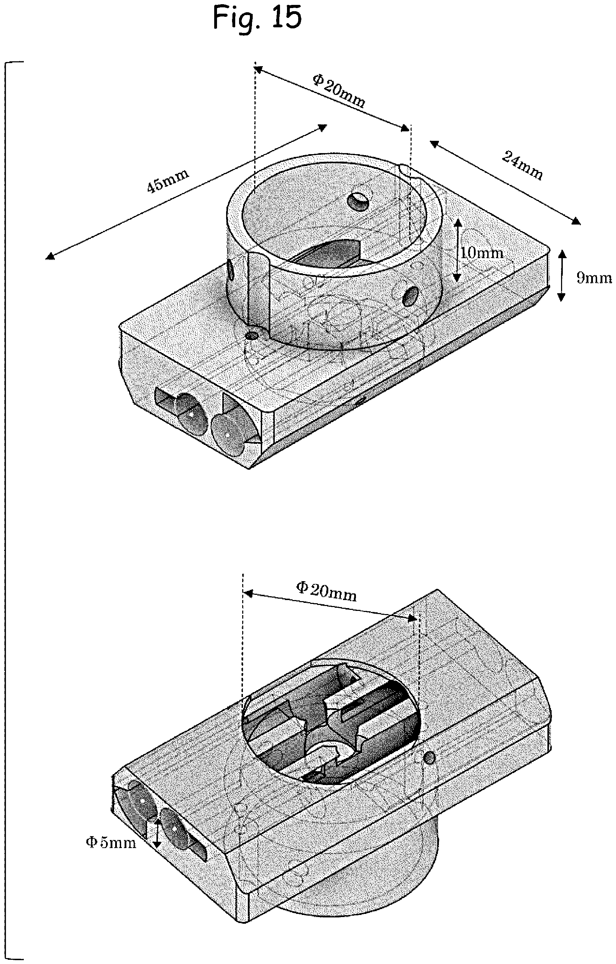

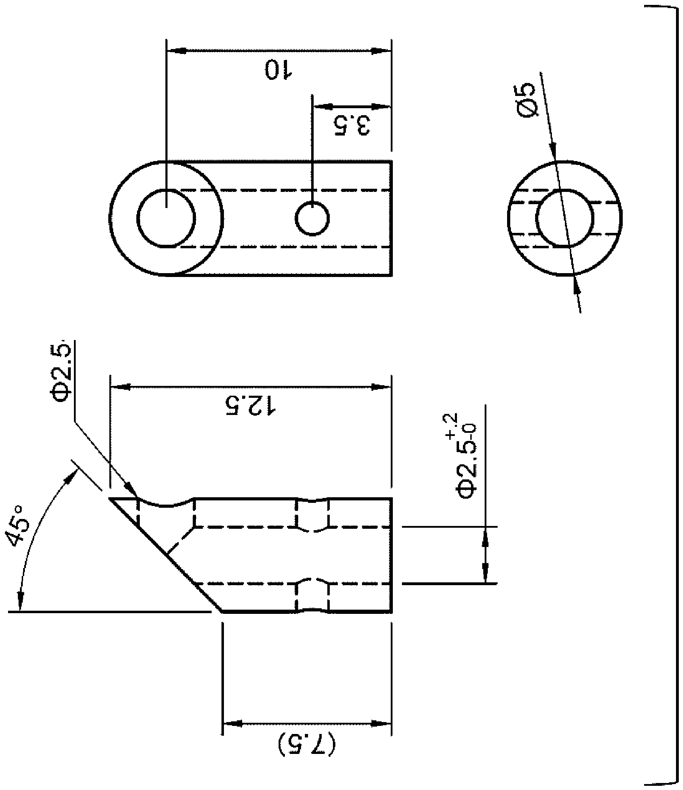

The present inventor actually performed eye fundus observation in a simulated eye and a pig eye using the intraocular illumination device 1 according to the above-described embodiment. The holder 12 of the intraocular illumination device 1 used was manufactured by a 3D printer, and specific dimensions thereof were as illustrated in Fig. 15. In addition, the cylindrical portion 110 of the illumination unit 11 of the intraocular illumination device 1 used was made of brass, and the specific dimensions thereof were as illustrated in Fig. 16. At the time of the eye fundus observation, a distance from a cornea 21 to a bottom of the holder 12 was 10 mm. Fig. 8 illustrates a fundus photograph taken when eye fundus observation was actually performed in the simulated eye (a diameter of an eyeball is 24 mm, and a diameter of the cornea is 12 mm). As illustrated in Fig. 8, by using the intraocular illumination device 1 according to the present embodiment, it has been confirmed that it is possible to realize intraocular illumination from the outside the eye having a sufficient light amount and range while using an existing microscope, and to perform eye fundus observation sufficient for surgery.

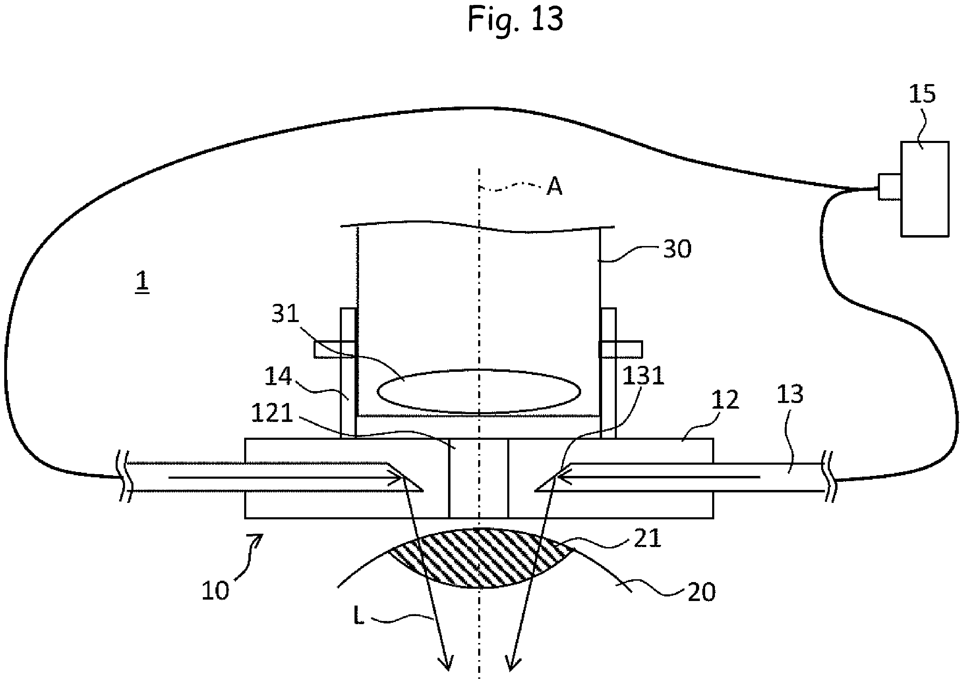

Note that, in the above-described embodiment, the reflecting portion provided at the tip portion of the fiber 13 and reflecting the light guided through the fiber 13 toward the inside of the eye 20 is configured by the mirror 111 of the illumination unit 11. However, the present disclosure is not limited to such an aspect, and for example, as illustrated in Fig. 13, an end surface 131 of the tip portion of the fiber 13 may be subjected to oblique polishing processing so as to reflect the light guided through the fiber 13 toward the inside of the eye 20. In this case, the reflecting portion provided at the tip portion of the fiber 13 and reflecting the light guided through the fiber 13 toward the inside of the eye 20 is configured by the end surface 131 of the tip portion of the fiber 13.

The present inventor obliquely polished the end surface of the tip portion of the SMA connector-attached multimode fiber (SI-MMF 400, NA 0.39) manufactured by Thorlabs by laser processing to produce a first sample having an end surface angle of 55.7° and a second sample having an end surface angle of 55.1°. Here, the end surface angle refers to an angle formed by a plane perpendicular to the optical axis of the fiber 13 and the end surface 131. Then, as a result of confirming the emission state of light from the tip portion of the fiber 13, it has been confirmed that about 65% of light guided through the fiber 13 can be reflected by the end surface 131 and emitted downward in both the first sample and the second sample, and intraocular illumination from the outside the eye having a sufficient light amount and range can be realized.

The description of the above-described embodiment and the disclosure of the drawings are merely examples for describing the invention described in the claims, and the invention described in the claims is not limited by the description of the above-described embodiment or the disclosure of the drawings. The components of the above-described embodiment can be arbitrarily combined without departing from the gist of the invention.

## Claims

1. An intraocular illumination device comprising:
a fiber that guides light from a light source; and
a holder that is disposed between an objective lens of a microscope and an eye during eye surgery or examination and supports a tip portion of the fiber, wherein
the tip portion of the fiber is provided with a reflecting portion that reflects the light guided through the fiber toward an inside of the eye.

2. The intraocular illumination device according to claim 1, wherein
an illumination unit including a mirror that reflects the light guided through the fiber toward the inside of the eye is attached to the tip portion of the fiber, and
the reflecting portion is configured by the mirror.

3. The intraocular illumination device according to claim 1, wherein
an end surface of the tip portion of the fiber is obliquely polished so as to reflect the light guided through the fiber toward the eye, and
the reflecting portion is configured by the end surface of the fiber.

4. The intraocular illumination device according to any one of claims 1 to 3, wherein
a thickness of the holder is 10 mm or less.

5. The intraocular illumination device according to any one of claims 1 to 4, wherein
the holder supports a plurality of the tip portions of the fibers.

6. The intraocular illumination device according to claim 5, wherein
a plurality of the reflecting portions provided at each of the plurality of tip portions are disposed in the holder so as to be arranged at equal intervals along a circumferential direction about an optical axis of the microscope.

7. The intraocular illumination device according to any one of claims 1 to 6, wherein
the holder is provided with an attachment portion attachable to a lens unit of the microscope.

8. The intraocular illumination device according to any one of claims 1 to 6, wherein
the holder is configured integrally with a lens unit of the microscope.

9. The intraocular illumination device according to any one of claims 1 to 8, wherein
a through-hole is formed in the holder coaxially with the optical axis of the microscope, a groove or hole is formed to extend outward from a vicinity of the through-hole in plan view, and the tip portion of the fiber is inserted into the groove or hole formed in the holder and supported.

10. The intraocular illumination device according to claim 9, wherein
the tip portion of the fiber is positionally adjustable along the groove or hole in a state of being supported by the holder.

11. The intraocular illumination device according to claim 10, wherein
the tip portion of the fiber is positionally adjusted along the groove or hole, so that the reflecting portion can be brought close to a range within 5 mm from the optical axis of the microscope.

12. The intraocular illumination device according to any one of claims 9 to 11, wherein
a number of the tip portions of the fibers is four, the holder is formed with four of the grooves or holes, and the four grooves or holes are formed to extend in directions of 0°, 90°, 180°, and 270°, respectively, in polar coordinates about the optical axis of the microscope.

13. The intraocular illumination device according to any one of claims 9 to 11, wherein
a number of the tip portions of the fibers is four, the holder is formed with four of the grooves or holes, a first groove or hole is formed to extend in a direction of 0° from a position of 0°, a second groove or hole is formed to extend in a direction of 180° from a position of 180°, a third groove or hole is formed to extend in a direction of 0° or 180° from a position of 90°, and a fourth groove or hole is formed to extend in a direction of 0° or 180° from a position of 270° in polar coordinates about the optical axis of the microscope.

14. The intraocular illumination device according to any one of claims 1 to 13, wherein
an NA of the fiber on an emitting side is 0.55 or less.

15. The intraocular illumination device according to any one of claims 1 to 14, wherein
the fiber is branched between the light source and the illumination unit.

16. The intraocular illumination device according to any one of claims 1 to 15, wherein
the light source is at least one of a xenon lamp, a halogen lamp, and an LED.

17. An intraocular illumination attachment disposed between an objective lens of a microscope and an eye during eye surgery or examination, the intraocular illumination attachment comprising:
an illumination unit attached to a tip portion of a fiber that guides light from a light source and including a mirror that reflects the light guided through the fiber into the eye; and
a holder that supports the illumination unit.

18. The intraocular illumination attachment according to claim 17, wherein
the intraocular illumination attachment is disposable.

19. An intraocular illumination device comprising:
the intraocular illumination attachment according to claim 17 or 18; and the fiber.

20. A microscope for eye surgery or examination comprising:
the intraocular illumination device according to any one of claims 1 to 16 and 19.
